# EUROPEAN PATENT APPLICATION

(11) **EP 4 772 210 A1**
(43) Date of publication of application: **08.07.2026**
(21) Application number: 24871740.7
(22) Date of filing: 02.09.2024
(51) Int. Cl.: A61M 5/142, A61M 5/145

(54) **LIQUID MEDICINE ADMINISTRATION SYSTEM**

(30) Priority: 29.09.2023 JP 2023170186
(71) Applicant: Terumo Kabushiki Kaisha, Tokyo 151-0072 (JP)
(72) Inventor: SEKIGUCHI, Shota, Ashigarakami-gun, Kanagawa 259-0151 (JP); UCHIYAMA, Joji, Ashigarakami-gun, Kanagawa 259-0151 (JP); YAMAZAKI, Tsuyoshi, Ashigarakami-gun, Kanagawa 259-0151 (JP)
(74) Representative: KIPA AB
(86) International application number: PCT/JP2024/031445
(87) International publication number: WO 2025/069940

(57) **Abstract**

A casing (120) of a drug solution administration apparatus (100) supports a lock member (260) for positioning and fixing the drug solution administration apparatus to the holder (20).
The lock member has an engagement convex portion (276) extending toward the holder.
The holder has an engagement portion (56) with which the engagement convex portion is detachably engaged.
The lock member in a state in which the engagement convex portion is engaged with the engagement portion is biased by an elastic body (290) in a direction in which the engagement convex portion is engaged with the engagement portion (toward an engagement direction (T1)).

## Description

### Technical Field

The present invention relates to a drug solution administration system for administering a drug solution to a living body.

### Background Art

A portable drug solution administration system includes a drug solution administration apparatus and a holder to which the drug solution administration apparatus is attached. When using a drug solution administration system, a user first fixes the holder to the body of a patient by attaching it with an adhesive. Next, the user causes a distal end of a cannula to protrude from the holder and indwell in the body of the patient, and further attaches the drug solution administration apparatus to the holder. Thereafter, the drug solution in a reservoir constituting the drug solution administration apparatus is discharged to the outside of the reservoir. The drug solution discharged from the reservoir is administered into the body of the patient through the cannula.

In Japanese Patent No. 6710056, the holder has a protrusion extending along an axial direction of the holder and a through hole. Meanwhile, the drug solution administration apparatus has a groove and a hook portion. When the drug solution administration apparatus is moved along the axial direction of the holder in order to attach the drug solution administration apparatus to the holder, the protrusion is elastically deformed in a width direction of the drug solution administration apparatus and engages with the groove, so that the drug solution administration apparatus is guided. When the hook portion is engaged with the through hole, the drug solution administration apparatus is stopped, and the attachment of the drug solution administration apparatus to the holder is completed.

### Citation List

### Patent Literature

PTL 1: Japanese Patent No. 6710056

### Summary of Invention

When the drug solution administration system is large, a patient to whom the drug solution administration system is fixed to the body feels the drug solution administration system as an obstacle. Therefore, miniaturization of the drug solution administration system is required.

An object of the invention is to solve the above-described problems.

(1) According to an aspect of the invention, there is provided a drug solution administration system including: a drug solution administration apparatus having a reservoir filled with a drug solution; and a holder to which the drug solution administration apparatus is detachably attached, in which a cannula to be punctured into a living body is provided in the holder.

The drug solution administration apparatus has a casing that accommodates the reservoir, a lock member supported by the casing and positions and fixes the drug solution administration apparatus to the holder, and an elastic body that biases the lock member. The casing has an apparatus-side base portion that faces the living body via the holder when the drug solution administration apparatus is attached to the holder. The lock member has an engagement convex portion extending toward the holder.

The holder has a holder-side base portion that holds the cannula, and an engagement portion with which the engagement convex portion detachably engages. The holder-side base portion has a first outer surface that faces the living body when the holder is fixed to the living body, and a first inner surface that is a back surface of the first outer surface and faces the apparatus-side base portion. The apparatus-side base portion has a second outer surface facing the first inner surface and a second inner surface that is a back surface of the second outer surface and faces the inside of the casing when the drug solution administration apparatus is attached to the holder.

A direction in which the second outer surface relatively moves along the first inner surface when the drug solution administration apparatus is attached to the holder is defined as an attachment direction. A direction opposite to the attachment direction and in which the second outer surface relatively moves along the first inner surface when the drug solution administration apparatus is detached from the holder is defined as a detachment direction. A direction along the attachment direction and the detachment direction is defined as an apparatus moving direction. A direction orthogonal to the apparatus moving direction and in which the second inner surface and the first outer surface face each other is defined as a thickness direction. One direction in the thickness direction from the second inner surface toward the first outer surface is defined as an engagement direction. The other direction in the thickness direction, which is a direction opposite to the engagement direction, is defined as an engagement release direction.

In the above configuration, the lock member is supported by the casing to be movable in the engagement direction and the engagement release direction. The elastic body biases the lock member in a state in which the engagement convex portion is engaged with the engagement portion toward the engagement direction.

The movement direction of the lock member is restricted to the engagement direction and the engagement release direction (thickness direction of the casing). That is, the lock member is prevented from moving in a direction (width direction of the casing) orthogonal to the apparatus moving direction and also orthogonal to the engagement direction and the engagement release direction. Therefore, it is possible to avoid an increase in the size of the casing along the width direction. Accordingly, it is possible to reduce the size of the drug solution administration apparatus.

The lock member is biased in the engagement direction by the elastic body, so that the engagement convex portion is prevented from coming off from the engagement portion. Therefore, even when a user or the like of the drug solution administration system unintentionally touches the casing, a state in which the drug solution administration apparatus is positioned and fixed to the holder is maintained. In other words, even when a force in a direction in which the drug solution administration apparatus is detached from the holder acts on the casing due to an unexpected situation, the drug solution administration apparatus is prevented from being detached from the holder.

(2) In the drug solution administration system according to Item (1), when the drug solution administration apparatus is attached to the holder, the holder has a holder-side guide portion configured to guide the engagement convex portion relatively moving along the attachment direction to the engagement portion. In this case, the holder-side guide portion may have a slope inclined toward the engagement release direction as approaching the engagement portion along the attachment direction.

When the engagement convex portion moves along the holder-side guide portion, the lock member moves relative to the casing in an engagement release direction in which the lock member is separated from the holder. Accordingly, the elastic body is compressed. Therefore, when the engagement convex portion reaches the engagement portion via the holder-side guide portion, the elastic body expands. The lock member is biased toward the holder by the expansion.

Therefore, when the engagement convex portion is detached from the engagement portion, the elastic body needs to be compressed. In other words, unless the elastic body is compressed, the engagement convex portion is continuously prevented from coming off from the engagement portion. Therefore, the drug solution administration apparatus is further prevented from being detached from the holder.

(3) In the drug solution administration system according to Item (1) or (2), the casing may have a casing-side guide portion configured to guide a movement of the lock member in the engagement direction or the engagement release direction.

The casing-side guide portion further restricts the movement of the lock member in a direction other than the engagement direction and the engagement release direction. Therefore, the casing is further prevented from increasing in size along the width direction.

(4) In the drug solution administration system according to any one of Items (1) to (3), the drug solution administration apparatus may have an unlocking member provided to be movable with respect to the casing, and the lock member may move in the engagement release direction based on a movement of the unlocking member, so that the engagement convex portion may be detached from the engagement portion.

The user can easily detach the drug solution administration apparatus from the holder by operating the unlocking member.

(5) In the drug solution administration system according to Item (4), the unlocking member may have a cam pin including a first cam surface, and the lock member may have a second cam surface with which the first cam surface slides, and the first cam surface and the second cam surface may convert a motion of the unlocking member moving relative to the casing in the detachment direction into a motion of the lock member moving in the engagement release direction.

By moving the unlocking member in the detachment direction in which the drug solution administration apparatus is detached from the holder, the engagement convex portion is detached from the engagement portion. That is, the detachment direction of the drug solution administration apparatus from the holder coincides with a direction in which the unlocking member is moved to detach the drug solution administration apparatus from the holder. Therefore, the user can easily understand the movement direction of the unlocking member for unlocking.

(6) In the drug solution administration system according to Item (5), each of the first cam surface and the second cam surface may be a slope that is inclined toward the engagement release direction from the detachment direction toward the attachment direction.

By moving the unlocking member in the detachment direction, the engagement convex portion of the lock member can be easily detached from the engagement portion.

(7) In the drug solution administration system according to any one of Items (1) to (6), the casing may have a base member including the apparatus-side base portion and a cover member interlocked to the base member. The base member may have a peripheral wall bent toward the engagement release direction with respect to the apparatus-side base portion. The cover member may have a cover wall covering the peripheral wall from outside. The cover wall may have an outer wall portion forming an outer surface of the cover wall, an inner wall portion located inward of the outer wall portion, and a pocket-shaped storage space formed between the inner wall portion and the outer wall portion. In this configuration, the lock member may be stored in the storage space.

According to the configuration, the lock member is located in the storage space between the inner wall portion and the outer wall portion. Therefore, the lock member is prevented from being exposed in the width direction of the casing. Accordingly, it is possible to reduce the size of the drug solution administration apparatus.

(8) In the drug solution administration system according to any one of Items (1) to (7), the lock member may have a main body portion, and a leg protruding from an end portion of the main body portion in the apparatus moving direction and extending in the engagement direction, and the engagement convex portion may be provided on the leg.

Since the leg extends in the engagement direction, the engagement convex portion can be provided at a position where the leg can easily engage with the engagement portion. That is, according to the configuration, the engagement convex portion easily engages with the engagement portion.

(9) In the drug solution administration system according to Item (8), the leg may have a first leg protruding from an end portion of the main body portion in the attachment direction and a second leg protruding from an end portion of the main body portion in the detachment direction. The elastic body may have a first elastic body that biases the first leg in the engagement direction, and a second elastic body that biases the second leg in the engagement direction. The engagement convex portion may be provided on the first leg or the second leg.

Since the first leg is biased by the first elastic body and the second leg is biased by the second elastic body, a biasing force of the first elastic body to the lock member and a biasing force of the second elastic body to the lock member can be balanced. Therefore, for example, when the engagement convex portion is guided by the holder-side guide portion, the lock member is prevented from being inclined with respect to the engagement direction or the engagement release direction. Therefore, the engagement convex portion is easily engaged with the engagement portion.

(10) In the drug solution administration system according to the item (8), the engagement convex portion may be located further on the attachment direction side than the main body portion and located further on the detachment direction side than the elastic body, in the apparatus moving direction.

According to the configuration, for example, the lock member whose leg is biased by the elastic body is prevented from rotating around the leg. That is, the posture of the lock member is stabilized. Therefore, the engagement convex portion can easily move toward the engagement portion. As a result, the engagement convex portion easily engages with the engagement portion.

According to the invention, it is possible to avoid an increase in the size of the casing constituting the drug solution administration apparatus. Accordingly, the drug solution administration apparatus can be miniaturized. The engagement convex portion of the lock member is engaged with the engagement portion of the holder, and the elastic body biases the lock member, thereby preventing the drug solution administration apparatus from being detached from the holder.

### Brief Description of Drawings

[FIG. 1] FIG. 1 is a schematic overall side view of a drug solution administration system viewed from a width direction.
[FIG. 2] FIG. 2 is a schematic overall perspective view of a holder constituting the drug solution administration system.
[FIG. 3] FIG. 3 is a schematic side cross-sectional view of the drug solution administration system viewed from the width direction.
[FIG. 4] FIG. 4 is a schematic plan view of the drug solution administration apparatus viewed from an engagement release direction.
[FIG. 5] FIG. 5 is a cross-sectional view taken along line V-V in FIG. 4.
[FIG. 6] FIG. 6 is a schematic perspective view of a base member constituting a casing of the drug solution administration apparatus.
[FIG. 7] FIG. 7 is a partial cross-sectional perspective view of a cover member constituting the casing of the drug solution administration apparatus.
[FIG. 8] FIG. 8 is a side cross-sectional view of a main part of the drug solution administration system.
[FIG. 9] FIG. 9 is a side cross-sectional view of the main part showing a state in which the drug solution administration apparatus is moved in an attachment direction from the state shown in FIG. 8.
[FIG. 10] FIG. 10 is a side cross-sectional view of the main part showing a state in which the drug solution administration apparatus is moved in the attachment direction from the state shown in FIG. 9.
[FIG. 11] FIG. 11 is a side cross-sectional view of a main part showing a state in which the drug solution administration apparatus moves in the attachment direction from the state shown in FIG. 10 and an engagement convex portion is engaged with the engagement portion.
[FIG. 12] FIG. 12 is a side cross-sectional view of the main part showing a state in which the engagement convex portion is detached from the engagement portion.

### Description of Embodiments

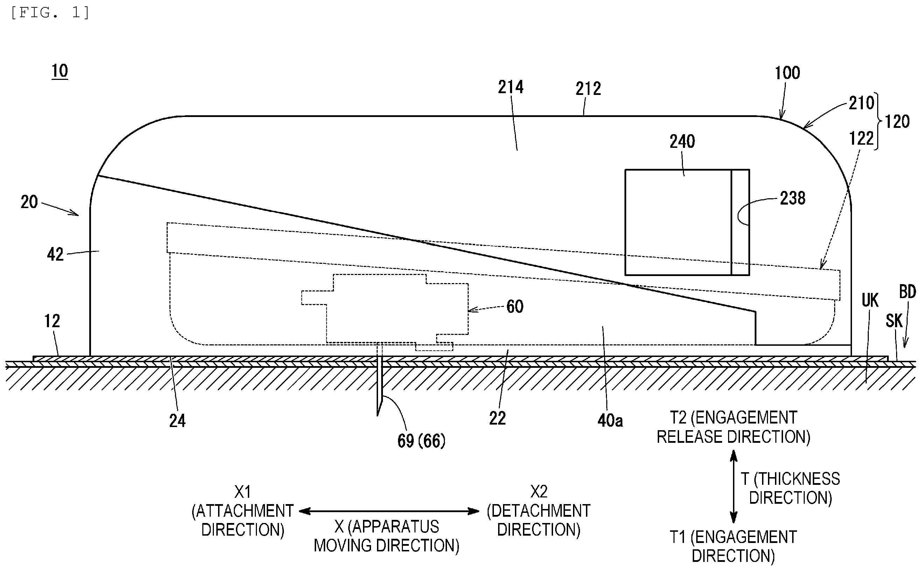

In the following description, a patient is exemplified as a living body. A "user" refers to a person who uses a drug solution administration system 10 shown in FIG. 1 by attaching it to a body BD of a living body. A typical example of the user is a patient, and the user is not limited to the patient.

FIG. 1 is a schematic overall side view of the drug solution administration system 10 viewed from a width direction W (to be described later). The drug solution administration system 10 is used by being fixed to the body BD of a patient. The drug solution administration system 10 is fixed to a skin SK of the patient by, for example, an adhesive sheet 12 provided on a first outer surface 24 of a holder 20 constituting the drug solution administration system 10.

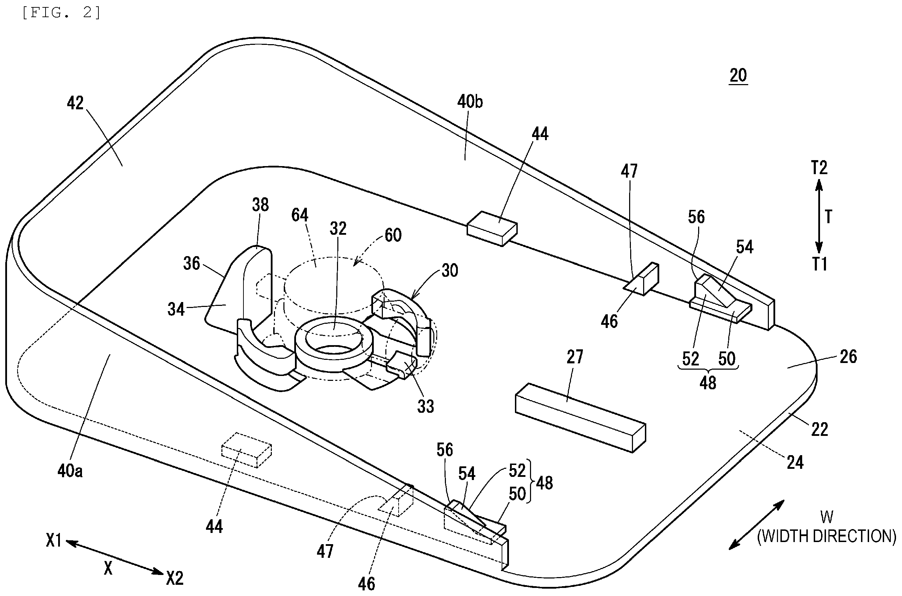

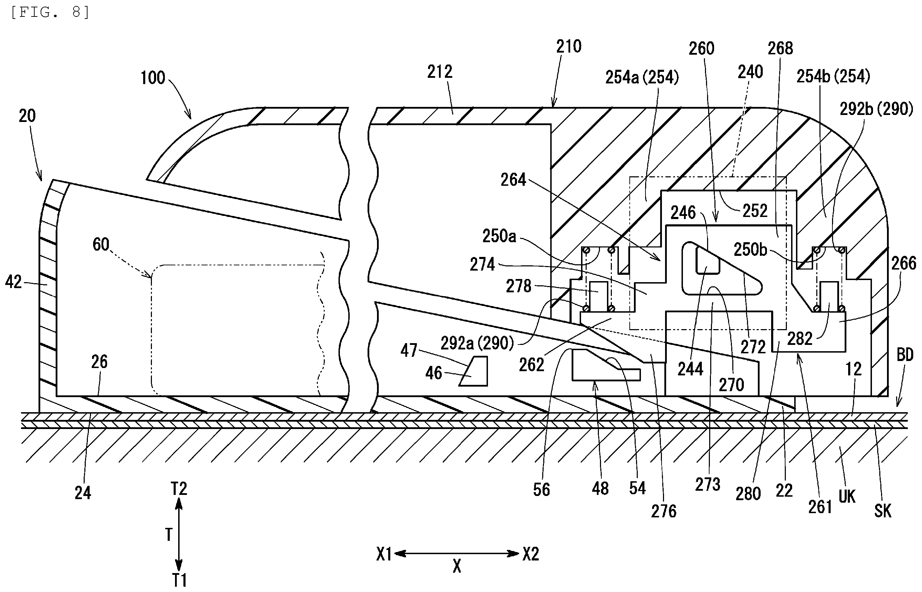

The drug solution administration system 10 has the holder 20 and a drug solution administration apparatus 100. As understood with reference to FIGS. 8 to 12, the drug solution administration apparatus 100 is detachably attached to the holder 20. When the drug solution administration apparatus 100 is attached to or detached from the holder 20, the drug solution administration apparatus 100 relatively moves along a first inner surface 26 of the holder 20 (see FIG. 2). A relative movement direction of the drug solution administration apparatus 100 on the first inner surface 26 when the drug solution administration apparatus 100 is attached to the holder 20 is a direction in which the drug solution administration apparatus 100 approaches an arch-shaped portion 42 of the holder 20. Hereinafter, the direction is referred to as an attachment direction X1. The relative movement direction of the drug solution administration apparatus 100 on the first inner surface 26 when the drug solution administration apparatus 100 is detached from the holder 20 is a direction in which the drug solution administration apparatus 100 is separated from the arch-shaped portion 42. Hereinafter, the direction is referred to as a detachment direction X2. The attachment direction X1 and the detachment direction X2 are opposite to each other.

A direction along the attachment direction X1 and the detachment direction X2 is referred to as an apparatus moving direction X, and a direction parallel to a surface on which the first inner surface 26 extends among directions orthogonal to the apparatus moving direction X is referred to as the width direction W. Further, a direction orthogonal to the apparatus moving direction X and the width direction W is referred to as a thickness direction T.

As shown in FIG. 2, the holder 20 has a holder-side base portion 22. The holder-side base portion 22 has the first outer surface 24 and the first inner surface 26. The adhesive sheet 12 is provided on the first outer surface 24. When the holder 20 is fixed to the skin SK of the patient via the adhesive sheet 12, the first outer surface 24 faces the skin SK (body BD). The first inner surface 26 is a surface (back surface) opposite to the first outer surface 24.

The thickness direction T is a direction in which the first outer surface 24 and a second inner surface 127 of an apparatus-side base portion 124 constituting a casing 120 face each other. Hereinafter, a direction from the second inner surface 127 toward the first outer surface 24 in the thickness direction T is referred to as an engagement direction T1. In the thickness direction T, a direction from the first outer surface 24 toward the second inner surface 127 is referred to as an engagement release direction T2. The engagement direction T1 and the engagement release direction T2 are opposite to each other.

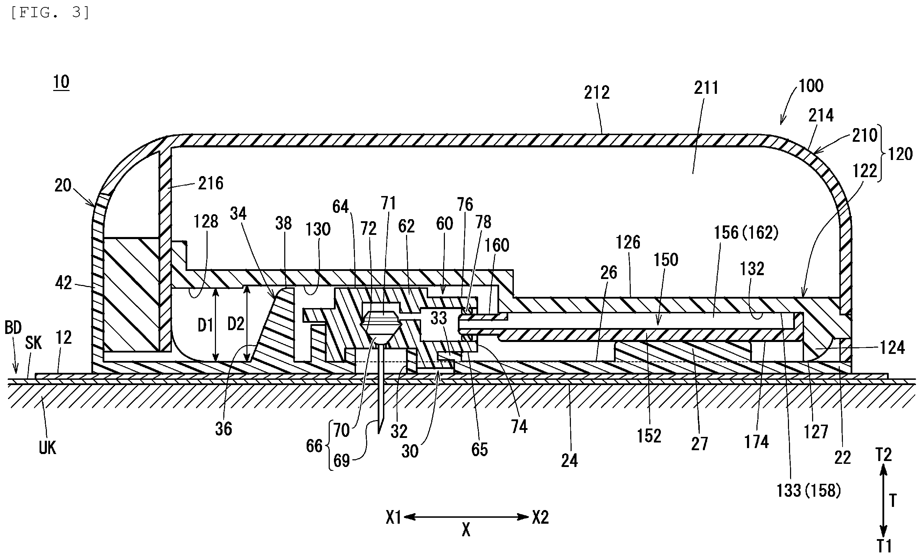

The holder-side base portion 22 has a unit holding portion 30 protruding from the first inner surface 26 in the engagement release direction T2. The unit holding portion 30 holds a cannula unit 60 to be described later. An insertion hole 32 penetrating along the thickness direction T of the holder-side base portion 22 is formed in the unit holding portion 30. The insertion hole 32 is located between both end portions of the holder-side base portion 22 in the apparatus moving direction X and between both end portions of the holder-side base portion 22 in the width direction W. A cannula 66 (see FIGS. 1 and 3) is inserted into the insertion hole 32. The unit holding portion 30 includes a hook 33. The hook 33 functions as a retainer when an unintended force is applied to the cannula unit 60 in the engagement release direction T2.

The holder-side base portion 22 has a protection portion 34 protruding from the first inner surface 26 in the engagement release direction T2. The protection portion 34 is provided further on the attachment direction X1 side than the unit holding portion 30 and extends along the thickness direction T of the holder-side base portion 22. A protruding direction of the protection portion 34 is the engagement release direction T2, which is one direction of the thickness direction T of the holder-side base portion 22, and is a direction opposite to the skin SK. In the protection portion 34, an end surface 36 on the attachment direction X1 is inclined toward the detachment direction X2 from the first inner surface 26 toward a top portion 38 of the protection portion 34. That is, the end surface 36 is a slope.

The holder-side base portion 22 further includes a ridge guide 27 protruding from the first inner surface 26 in the engagement release direction T2. The ridge guide 27 is a ridge protrusion extending in the apparatus moving direction X, and guides the movement of the drug solution administration apparatus 100 along the apparatus moving direction X when the drug solution administration apparatus 100 is attached to and detached from the holder 20.

The holder 20 has a first side portion 40a, a second side portion 40b, and the arch-shaped portion 42 that are continuous with the holder-side base portion 22. The first side portion 40a, the second side portion 40b, and the arch-shaped portion 42 protrude along the engagement release direction T2, similarly to the protection portion 34. When the drug solution administration apparatus 100 is attached to the holder 20, the first side portion 40a and the second side portion 40b sandwich the casing 120 in the width direction W. An end portion of the first side portion 40a on the attachment direction X1 and an end portion of the second side portion 40b on the attachment direction X1 are connected via the arch-shaped portion 42. The first side portion 40a, the second side portion 40b, and the arch-shaped portion 42 form an integrated standing wall portion.

Engagement ribs 44 are provided on an inner surface of the first side portion 40a. The engagement rib 44 protrudes inward in the width direction W. On the inner surface of the first side portion 40a, first guide ribs 46 and second guide ribs 48 are provided on the side further in the detachment direction X2 than the engagement ribs 44. The first guide rib 46 and the second guide rib 48 are separated from each other at a predetermined interval in the apparatus moving direction X.

The second guide rib 48 has a seat portion 50 protruding from the inner surface of the first side portion 40a in the width direction W, and a holder-side guide portion 52 extending from an upper surface of the seat portion 50 in the engagement release direction T2. In the holder 20 of the shown example, the holder-side guide portion 52 has a slope 54. The slope 54 is a surface inclined in a direction (engagement release direction T2) separated from the first inner surface 26 from the detachment direction X2 toward the attachment direction X1. A top portion of the holder-side guide portion 52 is located further in the engagement release direction T2 than a top portion of the first guide rib 46.

An end surface of the holder-side guide portion 52 on the attachment direction X1 extends linearly along the thickness direction T. An engagement convex portion 276 to be described later is detachably engaged with an upper portion of the end surface. That is, the upper portion of the end surface of the holder-side guide portion 52 on the attachment direction X1 forms the engagement portion 56.

Similarly, the engagement ribs 44, the first guide ribs 46, and the second guide ribs 48 are provided on an inner surface of the second side portion 40b. The holder-side guide portion 52 of the second guide rib 48 has the slope 54 inclined in a direction separated from the first inner surface 26 from the detachment direction X2 toward the attachment direction X1. The upper portion of the end surface of the holder-side guide portion 52 on the attachment direction X1 forms the engagement portion 56.

As shown in FIG. 3, the holder 20 is provided with the cannula unit 60. Specifically, the cannula unit 60 has a housing 62. When the drug solution is administered to the patient using the drug solution administration system 10, the housing 62 is attached to the unit holding portion 30 of the first inner surface 26 in the holder 20. The housing 62 attached to the unit holding portion 30 protrudes from the first inner surface 26 along the engagement release direction T2 (protruding direction of the protection portion 34). In the shown example, regarding a height from the first inner surface 26 in the engagement release direction T2, a height of the top portion 64, which is an outer surface of the housing 62 in the engagement release direction T2, is substantially the same as a height of the top portion 38 of the protection portion 34.

The housing 62 has a locking concave portion 65. When the user performs a predetermined operation using a puncture apparatus (not shown) attached to the holder 20 in advance, the housing 62 rotates. With the rotation, the hook 33 provided on the holder 20 engages with the locking concave portion 65. As a result, the cannula unit 60 is positioned and fixed to the unit holding portion 30.

The cannula unit 60 has a cannula 66 provided in the housing 62. The cannula 66 has a main body portion 69 located at the distal end and a flare portion 70 located at the proximal end. A fixing member 71 is attached to an upper portion of the flare portion 70. The fixing member 71 is fitted into a fitting concave portion 72 formed in the housing 62. By the fitting, the cannula 66 is held by the housing 62 via the fixing member 71. When a user performs a predetermined operation using a puncture tool, the fixing member 71 holding the cannula 66 approaches the unit holding portion 30, and the main body portion 69 of the cannula 66 is exposed from the insertion hole 32 together with the puncture needle of the puncture apparatus. The puncture needle and the main body portion 69 exposed from the insertion hole 32 puncture the skin SK (see FIGS. 1 and 3) of the patient.

The housing 62 has a first interlock portion 74. The first interlock portion 74 protrudes from a portion of the housing 62 facing the detachment direction X2. A protruding direction of the first interlock portion 74 is the detachment direction X2. A port 76 is formed inside the first interlock portion 74. The port 76 communicates with a lumen (not shown) of the cannula 66. A diaphragm 78 made of rubber or the like is disposed in the port 76. The diaphragm 78 may be, for example, a valve body with a slit. The diaphragm 78 is closed before the first interlock portion 74 is interlocked to a second interlock portion 160. That is, the first interlock portion 74 is sealed by the diaphragm 78. The diaphragm 78 protects the cannula 66 from an external environment by the sealing.

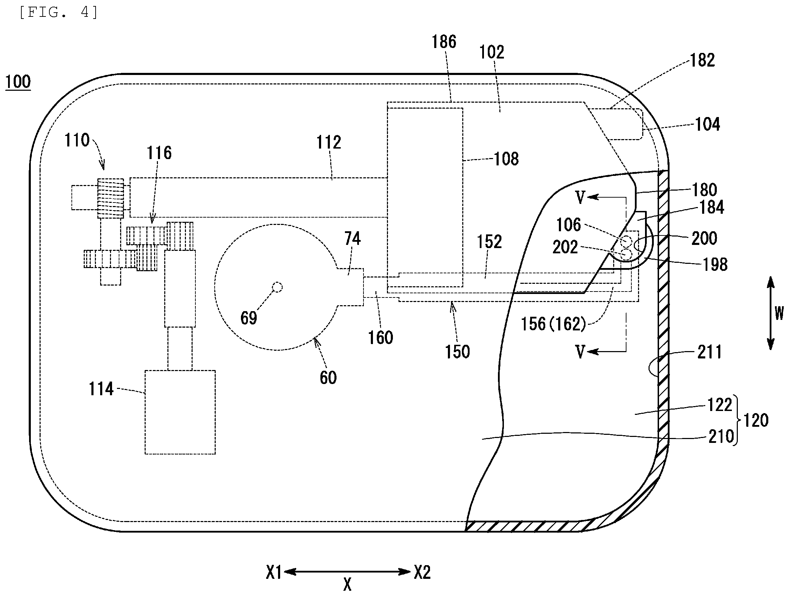

As shown in FIG. 4, the drug solution administration apparatus 100 has a reservoir 102 and the casing 120 that accommodates the reservoir 102. An axis of the reservoir 102 extends along the apparatus moving direction X of the drug solution administration apparatus 100.

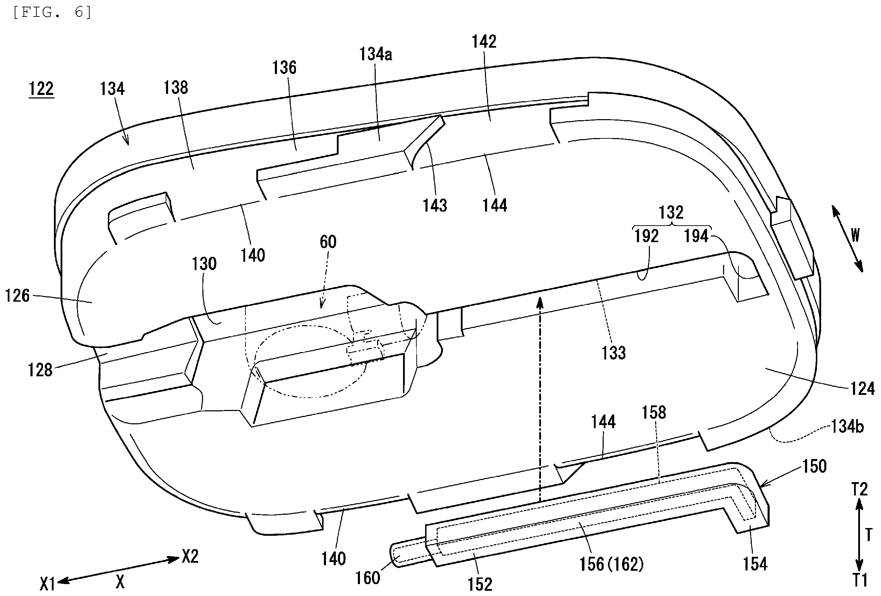

The casing 120 has a base member 122 and a cover member 210. As shown in FIGS. 3 and 6, the base member 122 has the apparatus-side base portion 124, and the apparatus-side base portion 124 has a second outer surface 126 and the second inner surface 127. The second outer surface 126 is a surface facing the first inner surface 26 of the holder 20 when the drug solution administration apparatus 100 is attached to the holder 20. The second inner surface 127 is a surface (back surface) opposite to the second outer surface 126 and faces the inside of the casing 120.

In the reservoir 102, an end portion (distal end) in the detachment direction X2 has a closed distal end portion 180. The distal end portion 180 has a first connection section 182 and a second connection section 184. The first connection section 182 extends in the detachment direction X2 from one end portion of the distal end portion 180 in the width direction W. The first connection section 182 has a drug solution inlet 104 at the distal end in the detachment direction X2. A drug solution is supplied into the reservoir 102 via the drug solution inlet 104 by the user performing a predetermined operation.

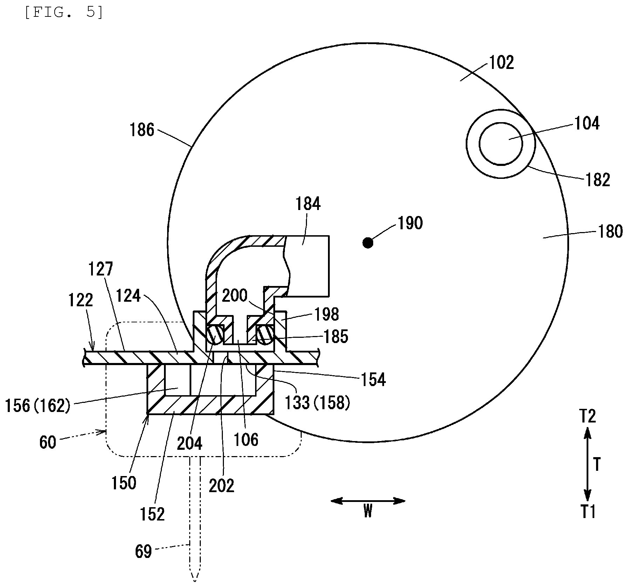

The second connection section 184 protrudes from the other end portion of the distal end portion 180 in the width direction W with respect to the end surface of the distal end portion 180. As shown in FIG. 5, when the second connection section 184 is viewed from the detachment direction X2, the second connection section 184 is located inward (toward a center in a cross section along the width direction W of the reservoir 102) of a curved side surface 186 to be described later. The second connection section 184 is an L-shaped flow path bent from the width direction W toward the engagement direction T1. The second connection section 184 has a projection portion 185 protruding in the engagement direction T1 and a drug solution outlet 106 formed in the projection portion 185. An outer diameter of the projection portion 185 is smaller than an outer diameter of the second connection section 184.

The apparatus-side base portion 124 has a tubular portion 198 on the second inner surface 127. The tubular portion 198 is formed by an annular wall extending from the second inner surface 127 in the engagement release direction T2. The tubular portion 198 has an interlock hole 200 therein. The projection portion 185 of the second connection section 184 is engaged with the tubular portion 198. Accordingly, the drug solution outlet 106 and the interlock hole 200 communicate with each other.

A flow hole 202 penetrates a bottom portion inside the tubular portion 198. As will be described later, the drug solution discharged from the reservoir 102 is supplied to a drug solution flow path 162 via the drug solution outlet 106 and the flow hole 202. Note that, an O-ring 204 is attached to an outer periphery of the projection portion 185. The O-ring 204 provides a liquid-tight seal between an inner peripheral surface of the interlock hole 200 and an outer peripheral surface of the projection portion 185. Accordingly, the drug solution is prevented from leaking from the interlock hole 200.

As shown in FIG. 5, the reservoir 102 has the curved side surface 186. The curved side surface 186 is curved to have a convex shape toward a flow path forming member 150 forming the drug solution flow path 162. The reservoir 102 has a central portion 190 located at the center in the engagement release direction T2. Note that, in the shown example, the reservoir 102 has a substantially perfect circular shape when viewed from the detachment direction X2, and the reservoir 102 may have a polygonal shape such as a quadrangular shape, an elliptical shape, or an oval shape when viewed from the detachment direction X2.

As shown in FIG. 4, inside the reservoir 102, a gasket 108 moves along the detachment direction X2, which is one direction of the apparatus moving direction X. A pump unit 110 is accommodated in the casing 120. The pump unit 110 has a plunger 112 that presses the gasket 108 and a motor 114 that drives the plunger 112. The motor 114 and the plunger 112 are interlocked via a plurality of gears 116. When the motor 114 is driven, the plunger 112 advances toward the drug solution outlet 106 located on the detachment direction X2. With this progress, the drug solution in the reservoir 102 is pushed by the gasket 108 and flows out from the drug solution outlet 106.

The casing 120 may be implemented to be separable into the base member 122 and the cover member 210. In this case, for example, in the pump unit 110, the reservoir 102, the plunger 112, and a part of the plurality of gears 116 are disposed on the base member 122, and the motor 114, the remaining part of the plurality of gears 116, an electronic substrate, and the like are disposed on the cover member 210. A battery (not shown) for driving the gears 116 is disposed on either the base member 122 or the cover member 210.

The base member 122 further has a peripheral wall 134 connected to the apparatus-side base portion 124 to be bent. The peripheral wall 134 is erected along the engagement release direction T2 from the peripheral edge portions of the second outer surface 126 and the second inner surface 127. The apparatus-side base portion 124 and the peripheral wall 134 form an internal space capable of accommodating components constituting the pump unit 110 inside the base member 122.

As shown in FIGS. 3 and 6, the second outer surface 126 of the apparatus-side base portion 124 has a semicircular hole-shaped preliminary concave portion 128, an accommodation concave portion 130, and a recessed groove 132. The preliminary concave portion 128, the accommodation concave portion 130, and the recessed groove 132 are arranged in this order from the attachment direction X1 toward the detachment direction X2, and are recessed in a direction separated from the first inner surface 26 of the holder 20 (engagement release direction T2). The preliminary concave portion 128, the accommodation concave portion 130, and the recessed groove 132 form a continuous space.

As shown in FIG. 3, an opening of the end portion of the preliminary concave portion 128 in the attachment direction X1 is covered with a shielding portion 216 of a cover wall 214 of the cover member 210 extending along the width direction W. Accordingly, the preliminary concave portion 128 opens only at a portion facing the first inner surface 26. The shielding portion 216 faces the arch-shaped portion 42 of the holder 20 in a state in which the drug solution administration apparatus 100 is attached to the holder 20. In the state in which the drug solution administration apparatus 100 is attached to the holder 20, the protection portion 34 is located between the shielding portion 216 and the housing 62 of the cannula unit 60 and is located in the accommodation concave portion 130. When the drug solution administration apparatus 100 is removed from the holder 20, the protection portion 34 relatively moves from the accommodation concave portion 130 toward the preliminary concave portion 128.

In the state in which the drug solution administration apparatus 100 is attached to the holder 20, the cannula unit 60 is accommodated in the accommodation concave portion 130. A distance (a depth D2) from the second outer surface 126 of the apparatus-side base portion 124 to a ceiling surface of the accommodation concave portion 130 is longer than a distance (a depth D1) from the second outer surface 126 of the apparatus-side base portion 124 to the ceiling surface of the preliminary concave portion 128.

As shown in FIG. 6, the recessed groove 132 has a substantially L shape. Specifically, the recessed groove 132 has a first linear groove 192 extending linearly along the apparatus moving direction X and a second linear groove 194 slightly extending linearly along the width direction W. The second linear groove 194 is connected to the end portion of the first linear groove 192 on the detachment direction X2 to be bent by approximately 90° toward the central axis of the reservoir 102 in a longitudinal direction. The second linear groove 194 is shorter than the first linear groove 192. As described above, the recessed groove 132 has a substantially L shape when the base member 122 is viewed from the engagement direction T1 toward the engagement release direction T2.

The recessed groove 132 has an in-groove ceiling surface 133. The in-groove ceiling surface 133 is an inner surface of the recessed groove 132 and is a part of the second outer surface 126. The in-groove ceiling surface 133 is a ceiling surface of the recessed groove 132.

The drug solution administration apparatus 100 has the flow path forming member 150 shown in FIGS. 3 to 6. When the drug solution administration apparatus 100 is viewed from the thickness direction T, the flow path forming member 150 has a substantially L shape corresponding to the shape of the recessed groove 132 (see FIGS. 4 and 6). Specifically, as shown in FIG. 6, the flow path forming member 150 has a first linear portion 152 extending linearly along the apparatus moving direction X and a second linear portion 154 slightly extending linearly along the width direction W. The second linear portion 154 is connected to an end portion of the first linear portion 152 on the detachment direction X2 to be bent by approximately 90° toward the reservoir 102. The second linear portion 154 is shorter than the first linear portion 152.

The flow path forming member 150 has a hollow inner portion 156 and an opening 158 (particularly, see FIG. 5). The opening 158 is formed in the flow path forming member 150, in a side portion facing the in-groove ceiling surface 133 of the recessed groove 132. The hollow inner portion 156 communicates with the opening 158. Therefore, when the flow path forming member 150 is cut along the width direction W and viewed from the apparatus moving direction X, the cross section is substantially U-shaped. The opening 158 opens in the engagement release direction T2. The hollow inner portion 156 corresponds to the shape of the flow path forming member 150 and is bent in a substantially L shape (see FIG. 6). The flow path forming member 150 further has the second interlock portion 160 at an end portion in the attachment direction X1. Note that, the opening 158 is not formed in the second interlock portion 160. That is, the second interlock portion 160 is a tubular portion having a lumen. The end surface of the second interlock portion 160 in the attachment direction X1 may be a plat surface.

A suitable material of the flow path forming member 150 is a resin. It is preferable that the flow path forming member 150 is transparent and the drug solution flowing through the hollow inner portion 156 (drug solution flow path 162) can be visually recognized from the outside of the flow path forming member 150. Examples of such a resin include polypropylene, polycarbonate, polyethylene, and an acrylonitrile-butadiene-styrene copolymer. However, the material of the flow path forming member 150 is not limited to the resin. The material of the flow path forming member 150 may be metal.

As shown in FIG. 3, the flow path forming member 150 is accommodated in the recessed groove 132. Specifically, the first linear portion 152 is accommodated in the first linear groove 192, and the second linear portion 154 is accommodated in the second linear groove 194 (see FIG. 6). A length of the flow path forming member 150 in the thickness direction T is shorter than a length (depth) of the recessed groove 132 in the thickness direction T. Therefore, in the thickness direction T, the entire flow path forming member 150 is accommodated in the recessed groove 132 (see FIG. 3). In other words, in the thickness direction T, the flow path forming member 150 is not exposed from the recessed groove 132. Therefore, as shown in FIG. 3, a guide concave portion 174 is formed between a surface of the second outer surface 126 located closest to the engagement direction T1 and a surface of the first linear portion 152 located closest to the engagement direction T1. The guide concave portion 174 extends along the apparatus moving direction X. In a state in which the drug solution administration apparatus 100 is disposed on the first inner surface 26 of the holder 20, the position of the guide concave portion 174 corresponds to the position of the ridge guide 27.

For example, as shown in FIGS. 3 and 5, the end surface of the flow path forming member 150 in the engagement release direction T2 is attached to the in-groove ceiling surface 133 of the recessed groove 132. The end surface and the in-groove ceiling surface 133 are joined by, for example, ultrasonic welding or an adhesive, and ultrasonic welding is most preferable. Accordingly, the opening 158 of the flow path forming member 150 is closed by the in-groove ceiling surface 133 (that is, the second outer surface 126). Accordingly, the drug solution flow path 162 is formed. Alternatively, a sealing material may be inserted between the end surface of the flow path forming member 150 and the in-groove ceiling surface 133.

As shown in FIGS. 4 and 5, when the drug solution administration apparatus 100 is viewed from the engagement release direction T2 toward the engagement direction T1, at least a part of the drug solution flow path 162 overlaps the position of the reservoir 102. That is, on the engagement direction T1 of the syringe-shaped reservoir 102, the drug solution flow path 162 is disposed along a gap generated along the longitudinal direction of the reservoir 102. The first linear portion 152 of the flow path forming member 150 and the drug solution flow path 162 are located on the engagement direction T1 with the base member 122 sandwiched therebetween in the width direction W of the reservoir 102. The length of the flow path forming member 150 in the apparatus moving direction X is slightly longer than the length of the reservoir 102 in the apparatus moving direction X (see FIG. 4). Therefore, the first linear portion 152 and the drug solution flow path 162, and the one end portion of the reservoir 102 in the width direction W overlap each other from the end portion of the distal end portion 180 of the reservoir 102 on the attachment direction X1 to the end portion of the reservoir 102 on the detachment direction X2.

As shown in FIG. 3, the second interlock portion 160 is exposed from the recessed groove 132 in the attachment direction X1 and is located in the accommodation concave portion 130. The second interlock portion 160 is interlocked to the first interlock portion 74. As a result, the drug solution administration apparatus 100 is attached to the holder 20. On the other hand, as shown in FIG. 5, the drug solution outlet 106 of the reservoir 102 and the hollow inner portion 156 of the flow path forming member 150 communicate with each other via the interlock hole 200 and the flow hole 202. As described above, the drug solution outlet 106 of the reservoir 102 and the port 76 of the housing 62 are connected via the drug solution flow path 162.

As shown in FIG. 6, the base member 122 has the peripheral wall 134. The peripheral wall 134 has a first side surface 134a and a second side surface 134b. When the drug solution administration apparatus 100 is attached to the holder 20, the first side surface 134a faces the inner surface of the first side portion 40a of the holder 20, and the second side surface 134b faces the inner surface of the second side portion 40b of the holder 20.

An engagement groove 136 is formed in the first side surface 134a. The engagement groove 136 is located slightly on the attachment direction X1 of a substantially central portion of the first side surface 134a in the apparatus moving direction X, and extends along the apparatus moving direction X. The engagement rib 44 is inserted into the engagement groove 136 to be movable along the apparatus moving direction X.

A first concave portion 138 extending in the engagement direction T1 is connected to an end portion of the engagement groove 136 on the attachment direction X1. The first concave portion 138 is formed as a notch recessed inward in the width direction W on the first side surface 134a when the base member 122 is viewed from the second outer surface 126. An end portion of the first concave portion 138 on the engagement direction T1 is continuous with a first insertion and removal opening 140. The first insertion and removal opening 140 is formed at a boundary between the apparatus-side base portion 124 and the first side surface 134a in the base member 122, and extends along the apparatus moving direction X. The first insertion and removal opening 140 opens a part of the first side surface 134a toward the second outer surface 126.

In the first side surface 134a, a second concave portion 142 is formed at a position further toward the detachment direction X2 than the engagement groove 136. The second concave portion 142 is a concave portion into which the first guide rib 46 and the second guide rib 48 are inserted. The second concave portion 142 is formed by the first side surface 134a being recessed inward in the width direction W. Accordingly, the first guide rib 46 and the second guide rib 48 are prevented from being interfered with the first side surface 134a. In the second concave portion 142, a second insertion and removal opening 144 is connected to an end portion on the engagement direction T1. The second insertion and removal opening 144 is formed at a boundary between the second outer surface 126 and the first side surface 134a of the base member 122, and extends along the apparatus moving direction X. The second insertion and removal opening 144 is formed as a notch recessed inward in the width direction W on the first side surface 134a when the base member 122 is viewed from the second outer surface 126. The second insertion and removal opening 144 opens a part of the first side surface 134a toward the second outer surface 126.

The second side surface 134b is also provided with components similar to those described above at a position where a symmetry line passing through the center of the casing 120 in the width direction W and parallel to the apparatus moving direction X is a symmetry center. That is, the second side surface 134b is also provided with the engagement groove 136, the first concave portion 138, the first insertion and removal opening 140, the second concave portion 142, and the second insertion and removal opening 144. As described above, in the holder 20, the engagement ribs 44, the first guide ribs 46, and the second guide ribs 48 are provided at positions corresponding to the respective components of the second side surface 134b. In this way, in the first side surface 134a and the second side surface 134b, by arranging the components that play the same role at positions symmetrical to each other in the apparatus moving direction X, it is possible to stably perform an attachment and detachment operation of the drug solution administration apparatus 100 with respect to the holder 20.

As shown in FIG. 1, the cover member 210 is interlocked to the base member 122 to cover the entire end portion of the base member 122 on the engagement release direction T2. Accordingly, as shown in FIGS. 3 and 4, an inner chamber 211 is formed by the cover member 210 and the base member 122. The reservoir 102 and the pump unit 110 are accommodated in the inner chamber 211 (see FIG. 4).

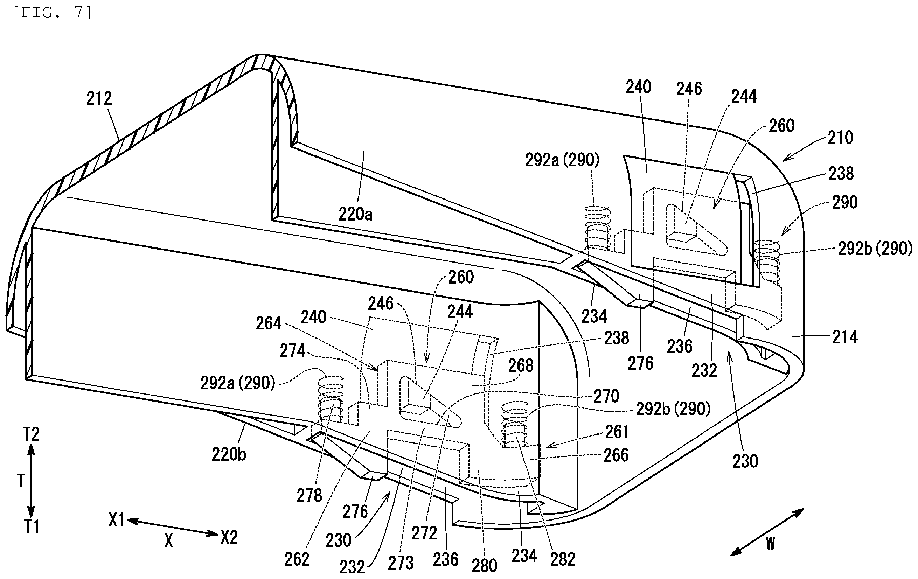

As shown in FIG. 7, the cover member 210 has a ceiling portion 212 extending along the apparatus moving direction X and the width direction W, and the cover wall 214 bent from the ceiling portion 212 toward the engagement direction T1. The cover wall 214 covers the peripheral wall 134 of the base member 122 from the outside. As described above, the shielding portion 216 (see FIG. 3) of the cover wall 214 constitutes an end portion of the preliminary concave portion 128 in the attachment direction X1.

Hereinafter, a structure for locking the drug solution administration apparatus 100 to the holder 20 will be described more specifically. Although a locking mechanism such as an unlocking member 240 is provided on two side surfaces facing the width direction W in the drug solution administration apparatus 100 and the holder 20, one side surface of the two side surfaces will be described in order to simplify the description and facilitate understanding.

As shown in FIG. 7, the cover wall 214 further has a first cover portion 220a located outside the first side surface 134a of the peripheral wall 134 and a second cover portion 220b located outside the second side surface 134b of the peripheral wall 134. A portion of the first cover portion 220a close to the end portion on the detachment direction X2 is a double wall portion 230. Specifically, the double wall portion 230 has an outer wall portion 232, an inner wall portion 234, and a storage space 236. An outer surface of the outer wall portion 232 is a part of an outer surface of the first cover portion 220a. That is, the outer surface of the outer wall portion 232 forms the outer surface of the cover wall 214. The inner wall portion 234 is located inward of the outer wall portion 232. The outer wall portion 232 and the inner wall portion 234 are slightly separated from each other by a predetermined interval. The separation forms a pocket-shaped storage space 236 between the outer wall portion 232 and the inner wall portion 234.

The double wall portion 230 of the first cover portion 220a has an insertion opening 238 penetrating from the outer surface of the outer wall portion 232 to the storage space 236. The insertion opening 238 has a substantially rectangular shape in which two sides extending along the apparatus moving direction X are slightly longer than two sides extending along the thickness direction T. The unlocking member 240 is inserted into the insertion opening 238.

The unlocking member 240 is a substantially square plate-shaped member, and has engagement claws (not shown) at an end portion on the engagement direction T1 and an end portion on the engagement release direction T2. The engagement claws are hooked on the inner surface of the outer wall portion 232 in the vicinity of the insertion opening 238 on the engagement direction T1 and in the vicinity of the insertion opening 238 on the engagement release direction T2. The unlocking member 240 is slidable in the insertion opening 238 along the apparatus moving direction X by a user operation.

The unlocking member 240 has a cam pin 244 provided on a surface facing inward in the width direction W. The cam pin 244 has a first cam surface 246. The first cam surface 246 is a slope inclined toward the engagement release direction T2 as the first cam surface progresses from the detachment direction X2 toward the attachment direction X1.

The description will be made with reference to the second cover portion 220b shown in FIG. 8. The storage space 236 has a first sub-storage portion 250a, a main storage portion 252, and a second sub-storage portion 250b in order from the attachment direction X1 to the detachment direction X2. The first sub-storage portion 250a and the second sub-storage portion 250b are inner chambers shaped like hollowed-out cylinders. The cover member 210 has a casing-side guide portion 254 provided on the outer wall portion 232. The casing-side guide portion 254 has a first casing-side guide portion 254a and a second casing-side guide portion 254b. The first casing-side guide portion 254a extends from a corner portion on the inner edge of the insertion opening 238, located on the attachment direction X1 and the engagement release direction T2, toward the engagement direction T1. The second casing-side guide portion 254b extends from the corner portion on the inner edge of the insertion opening 238, located on the detachment direction X2 and the engagement release direction T2, toward the engagement direction T1. The first casing-side guide portion 254a and the second casing-side guide portion 254b are parallel to each other.

The lock member 260 is stored in the storage space 236. As shown in FIG. 7, the lock member 260 integrally has a leg 261 and a main body portion 264. The leg 261 has a first leg 262 and a second leg 266. The first leg 262, the main body portion 264, and the second leg 266 are arranged in this order from the attachment direction X1 toward the detachment direction X2.

The main body portion 264 includes a head portion 268 having a substantially rectangular shape. A cam hole 270 into which the cam pin 244 of the unlocking member 240 is inserted is formed in the main body portion 264. The cam hole 270 has a second cam surface 272 with which the first cam surface 246 slides. The second cam surface 272 is a slope parallel to the first cam surface 246. That is, similarly to the first cam surface 246, the second cam surface 272 is a slope that is inclined toward the engagement release direction T2 from the detachment direction X2 toward the attachment direction X1.

The main body portion 264 has a reinforcing portion 273. The reinforcing portion 273 is located further on the engagement direction T1 side than the cam hole 270 and extends along the apparatus moving direction X. The reinforcing portion 273 improves rigidity of the lock member 260. However, the reinforcing portion 273 may be omitted. In this case, the cam hole 270 has an opening on the engagement direction T1.

The main body portion 264 has a protruding end portion 274 that protrudes further in the attachment direction X1 than the head portion 268. The protruding end portion 274 is located further on the engagement direction T1 side than the head portion 268. The first leg 262 is integrally interlocked to the protruding end portion 274. Specifically, the first leg 262 has a substantially right triangular shape. In the first leg 262, one side forming a top portion having a substantially right angle is interlocked to a surface facing the engagement direction T1 at an end portion of the protruding end portion 274 on the attachment direction X1. Accordingly, the first leg 262 protrudes from the end portion of the main body portion 264 on the attachment direction X1.

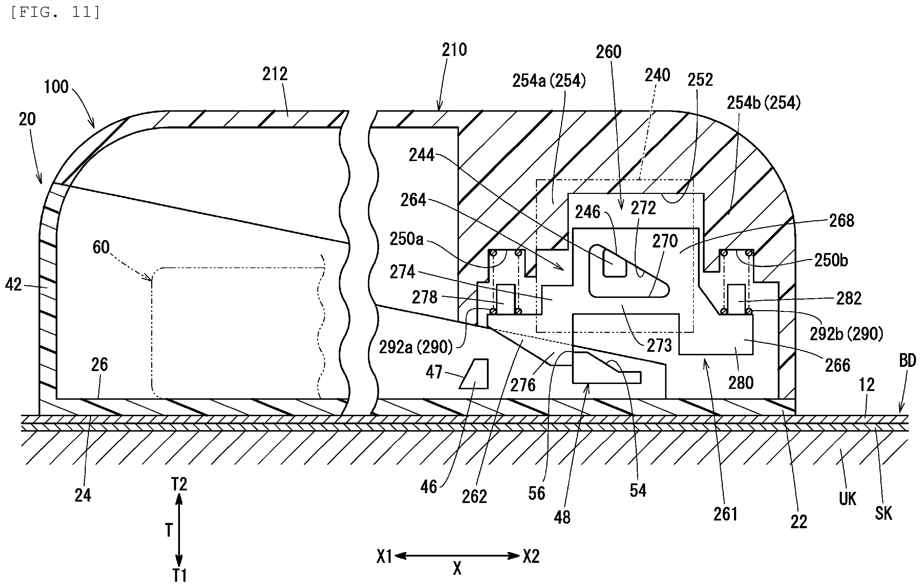

The first leg 262 has a convex end portion protruding in the engagement direction T1. The end portion is the engagement convex portion 276. That is, the first leg 262 has the engagement convex portion 276 at the end portion facing the engagement direction T1. As will be described later, when the drug solution administration apparatus 100 is attached to the holder 20, the engagement convex portion 276 engages with the engagement portion 56 of the holder 20 (see FIG. 11).

As shown in FIGS. 7 and 8, in the first leg 262, a first columnar portion 278 is provided on one side exposed from the protruding end portion 274. The first columnar portion 278 extends in the engagement release direction T2. The first columnar portion 278 is inserted into an end portion of a first elastic body 292a, which constitutes the elastic body 290, on the engagement direction T1. In the shown example, the first elastic body 292a is a coil spring. However, the first elastic body 292a is not particularly limited to the coil spring as long as the elastic body is an elastic object.

The main body portion 264 has an extending portion 280 extending in the engagement direction T1 from an end portion of the head portion 268 on the engagement direction T1. The second leg 266 protrudes in the detachment direction X2 from end surfaces of the head portion 268 and the extending portion 280 on the detachment direction X2. The second leg 266 has a substantially trapezoidal shape.

A second columnar portion 282 is provided at an end portion of the second leg 266 on the detachment direction X2. The second columnar portion 282 extends in the engagement release direction T2. The second columnar portion 282 is inserted into an end portion on the engagement direction T1 of a second elastic body 292b constituting the elastic body 290. In the shown example, the second elastic body 292b is a coil spring. However, the second elastic body 292b is not particularly limited to the coil spring as long as the elastic body is an elastic object.

As shown in FIG. 8, the main body portion 264 is stored in the main storage portion 252 of the storage space 236. In the main storage portion 252, the head portion 268 of the main body portion 264 is located between the first casing-side guide portion 254a and the second casing-side guide portion 254b in the apparatus moving direction X. The first columnar portion 278 is stored in the first sub-storage portion 250a together with the first elastic body 292a. The end portion of the first elastic body 292a on the engagement release direction T2 is attached to the inner surface of the ceiling portion 212 of the cover member 210. Therefore, the first elastic body 292a biases the first leg 262 in the engagement direction T1. The second columnar portion 282 is stored in the second sub-storage portion 250b together with the second elastic body 292b. The end portion of the second elastic body 292b on the engagement release direction T2 is attached to the inner surface of the ceiling portion 212 of the cover member 210. Therefore, the second elastic body 292b biases the second leg 266 in the engagement direction T1.

As understood from the above positional relationship, in this aspect, the engagement convex portion 276 is located further on the attachment direction X1 side than the main storage portion 252 and the main body portion 264, in the apparatus moving direction X. In addition, the engagement convex portion 276 is located further on the detachment direction X2 side than the first elastic body 292a, which constitutes the elastic body 290, in the apparatus moving direction X.

Note that, in the lock member 260, one of the first leg 262 and the second leg 266 may be omitted. For example, the second leg 266 and the second elastic body 292b may be omitted from the above configuration. Alternatively, the first leg 262 and the first elastic body 292a may be omitted from the above configuration. In the above configuration as well, the engagement convex portion 276 is detachably engaged with the engagement portion 56.

Similarly, in the first cover portion 220a, the lock member 260 is stored in the storage space 236 of the double wall portion 230 in a state of being supported by the cover member 210 to be movable in the engagement direction T1 and the engagement release direction T2. However, the lock member 260 may be omitted from one of the first cover portion 220a and the second cover portion 220b. In this case, in the holder 20, the second guide rib 48 of one of the first side portion 40a and the second side portion 40b may be omitted.

The drug solution administration system 10 is used as follows. First, the user fills the reservoir 102 in the base member 122 with the drug solution via the drug solution inlet 104 using, for example, a predetermined drug solution filling apparatus.

Next, the user pushes the plunger 112 toward the distal end of the reservoir 102 using a coin or the like in a state in which the drug solution outlet 106 of the reservoir 102 faces upward. Accordingly, as the plunger 112 moves in the detachment direction X2, most of the air in the reservoir 102 is pushed out by the gasket 108. Thereafter, the user attaches the cover member 210 to the base member 122.

Next, the user operates a remote controller (not shown) to perform a priming operation of filling the drug solution flow path 162 with the drug solution. Therefore, the motor 114 is driven. Accordingly, the drug solution in the reservoir 102 pressed by the gasket 108 and the air remaining in the drug solution flow path 162 and the like are discharged from the drug solution outlet 106. After moving to the interlock hole 200, the drug solution moves to the hollow inner portion 156 of the flow path forming member 150 via the flow hole 202 and the opening 158. As described above, the drug solution flows into the drug solution flow path 162. Since the flow path forming member 150 has a substantially L shape and the first linear portion 152 extends in the attachment direction X1, a flow direction of the drug solution in the drug solution flow path 162 is changed from the width direction W to the attachment direction X1. As described above, since the flow path forming member 150 has a substantially L shape, it is easy to change the direction of the drug solution pushed out from the reservoir 102 in the detachment direction X2 to the attachment direction X1 which is a direction opposite to the detachment direction X2.

Thereafter, the drug solution moves in the attachment direction X1 along the drug solution flow path 162. A small amount of the drug solution that reaches the second interlock portion 160 is discharged from a port of the second interlock portion 160. Accordingly, before the flow of the drug solution is started, the user can check that the air present inside the second connection section 184 and extending to the port of the second interlock portion 160 is pushed out by the drug solution, and that the drug solution flow path 162 is now filled with the drug solution. In this case, the user finishes pressing the drug solution by the plunger 112.

When the flow path forming member 150 is transparent, the user can see the drug solution flow path 162 from the outside of the flow path forming member 150. Therefore, for example, the user can quickly visually check whether a foreign matter is mixed in the drug solution in the drug solution flow path 162. Alternatively, the user can quickly visually check whether the drug solution in the drug solution flow path 162 bites air (whether air bubbles are present).

On the other hand, as shown in FIG. 8, the user fixes the holder 20 to the skin SK of a predetermined site of a patient via the adhesive sheet 12. Examples of the predetermined site include the abdomen. At this time, the cannula unit 60 (see FIGS. 2 and 3) is not held by the unit holding portion 30 of the holder 20. Next, the user performs a predetermined operation using a puncture apparatus (not shown). Accordingly, a puncture needle of the puncture apparatus and the main body portion 69 of the cannula 66 pass through the insertion hole 32 and puncture the skin SK of the patient. When the user rotates the housing 62 together with the puncture apparatus, the cannula unit 60 approaches the unit holding portion 30, and the hook 33 of the holder 20 engages with the locking concave portion 65 of the housing 62.

Accordingly, the cannula unit 60 is positioned and fixed to the holder 20. Based on this, a state in which the main body portion 69 of the cannula 66 is punctured into a subcutaneous tissue UK of the patient is maintained. That is, the main body portion 69 of the cannula 66 is indwelled in the subcutaneous tissue UK of the patient. Thereafter, the user removes the puncture apparatus from the holder 20. With this operation, only the puncture needle is removed from the subcutaneous tissue UK. Therefore, only the cannula 66 is indwelled in the subcutaneous tissue UK.

The user attaches, to the holder 20, the drug solution administration apparatus 100 in which the absence of air bubbles in the drug solution in the drug solution flow path 162 (particularly, see FIG. 6) is checked by visual inspection.

### [First Step]

In a process of attaching the drug solution administration apparatus 100 to the holder 20, as shown in FIG. 8, the user attaches the second outer surface 126 of the apparatus-side base portion 124 of the base member 122 to the first inner surface 26 of the holder 20 at a position where the protection portion 34 and the cannula unit 60 are accommodated in the accommodation concave portion 130 (see FIG. 3). At this time, the engagement rib 44 (see FIG. 2) is inserted into the engagement groove 136 via the first insertion and removal opening 140 (see FIG. 6). The first guide ribs 46 (see FIG. 2) and the second guide ribs 48 are inserted into the second concave portion 142 via the second insertion and removal opening 144 (see FIG. 6). The slope 47 (see FIG. 2) of the first guide rib 46 guides the slope 143 (see FIG. 6) of the second concave portion 142. Further, an upper portion of the ridge guide 27 (see FIG. 3) is inserted into the guide concave portion 174.

As shown in FIG. 8, at this time, in a side view from the width direction W, the end portion of the drug solution administration apparatus 100 on the detachment direction X2 side protrudes from the first inner surface 26 of the holder 20 to the detachment direction X2, and other portions of the drug solution administration apparatus 100 are placed on the first inner surface 26. At this stage, the first interlock portion 74 (see FIG. 3) of the housing 62 and the second interlock portion 160 of the drug solution administration apparatus 100 are not yet interlocked. The engagement convex portion 276 is separated from the holder-side guide portion 52 (the slope 54) constituting the second guide rib 48 on the detachment direction X2, and is not yet engaged with the engagement portion 56. Therefore, the drug solution administration apparatus 100 is not attached to the holder 20.

### [Second Step]

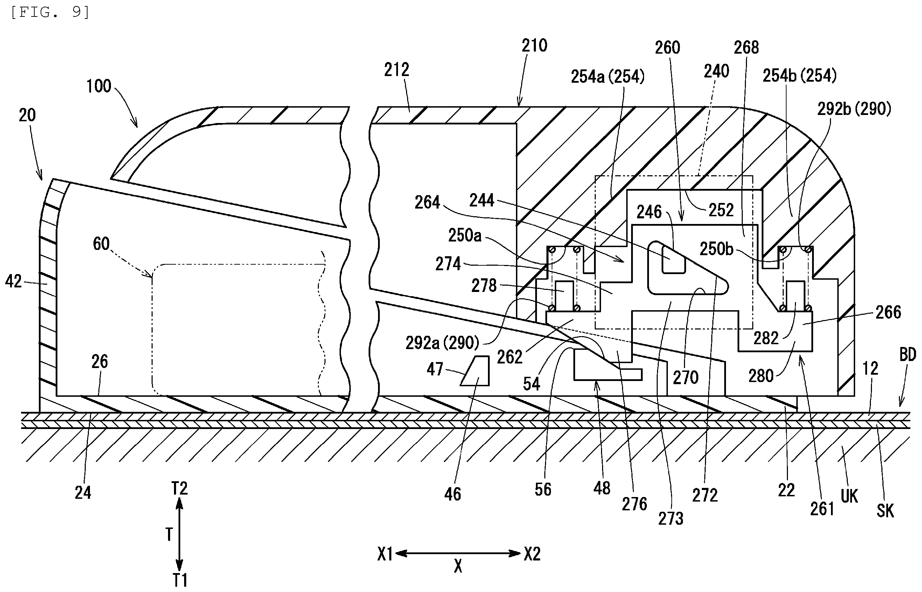

From this state, as shown in FIG. 9, the user slides the drug solution administration apparatus 100 toward the attachment direction X1 on the holder 20. The drug solution administration apparatus 100 moves relative to the holder 20 in the attachment direction X1 along the first inner surface 26. In the relative movement, the drug solution administration apparatus 100 is guided by the ridge guide 27 (see FIG. 3). Therefore, when the drug solution administration apparatus 100 is attached to the holder 20, the user can stably slide the drug solution administration apparatus 100 on the holder 20. The same applies to an attachment and detachment operation of the drug solution administration apparatus 100 from the holder 20.

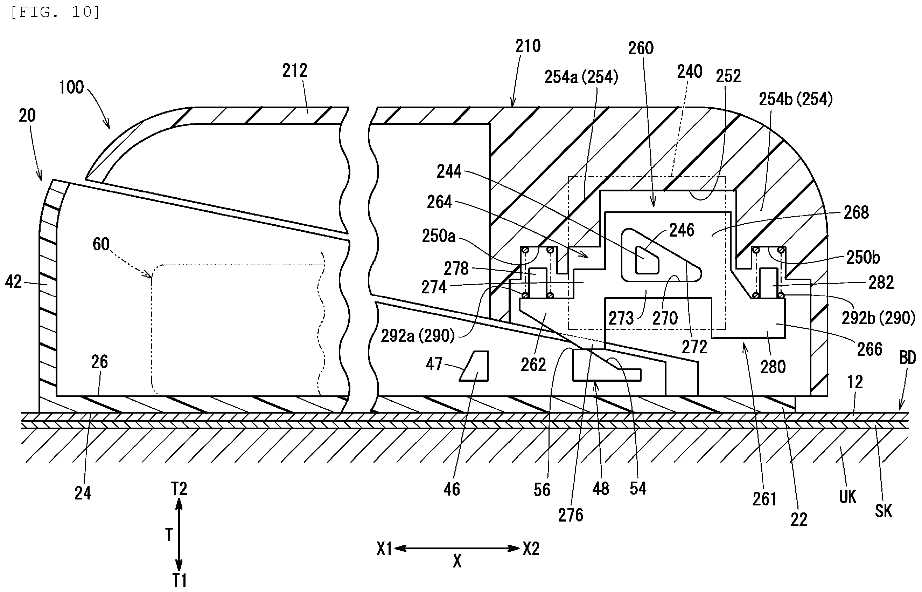

As the drug solution administration apparatus 100 relatively moves in the attachment direction X1 as described above, the lock member 260 also relatively moves in the attachment direction X1. Due to the movement, as shown in FIG. 9, the engagement convex portion 276 is attached to the holder-side guide portion 52 (the slope 54) . As shown in FIG. 10, when the drug solution administration apparatus 100 further relatively moves in the attachment direction X1, the engagement convex portion 276 receives a reaction force from the slope 54 and is pressed in the engagement release direction T2. Based on this, as can be understood by comparing FIGS. 9 and 10, the lock member 260 gradually moves in the engagement release direction T2 as the lock member relatively moves in the attachment direction X1. In the movement, the head portion 268 of the main body portion 264 is guided by the first casing-side guide portion 254a and the second casing-side guide portion 254b. As the lock member 260 moves in the engagement release direction T2, the first elastic body 292a and the second elastic body 292b are compressed.

### [Third Step]

As shown in FIG. 11, the user further slides the drug solution administration apparatus 100 on the holder 20 in the attachment direction X1. As a result, the first interlock portion 74 of the housing 62 and the second interlock portion 160 of the flow path forming member 150 are interlocked to each other (see FIG. 3). Accordingly, the drug solution outlet 106 of the reservoir 102 and the port 76 of the housing 62 are connected via the drug solution flow path 162 (the flow path forming member 150), and the drug solution administration apparatus 100 is attached to the holder 20.

At substantially the same time, as shown in FIG. 11, the engagement convex portion 276 passes through the holder-side guide portion 52 and reaches the engagement portion 56. At this time, the compressed first elastic body 292a and second elastic body 292b expand. The stretched first elastic body 292a and second elastic body 292b bias the lock member 260 in the engagement direction T1. Due to the biasing, the lock member 260 moves in the engagement direction T1. As a result, the engagement convex portion 276 engages with the engagement portion 56. The first elastic body 292a and the second elastic body 292b bias the lock member 260 in the engagement direction T1 even after the engagement convex portion 276 is engaged with the engagement portion 56. Accordingly, the engagement convex portion 276 is prevented from coming off the engagement portion 56. As a result, the drug solution administration apparatus 100 is prevented from moving in the detachment direction X2 on the holder 20.

The engagement ribs 44 (see FIG. 2) engage with the engagement grooves 136 (see FIG. 6). When the engagement convex portion 276 engages with the engagement portion 56 and the engagement rib 44 engages with the engagement groove 136, the drug solution administration apparatus 100 is positioned and fixed to the holder 20 in a state in which the second interlock portion 160 is interlocked to the first interlock portion 74. That is, the drug solution administration apparatus 100 is prevented from falling off from the holder 20.

### [Fourth Step]

Next, the user operates the remote controller. Accordingly, the motor 114 (see FIG. 4) is driven, and the plunger 112 and the gasket 108 move to the detachment direction X2. As a result, the drug solution in the reservoir 102 is pushed by the gasket 108 and discharged from the drug solution outlet 106. The drug solution moves in the attachment direction X1 along the drug solution flow path 162 as described above. Next, the drug solution flows from the second interlock portion 160 toward the first interlock portion 74, and then flows through the lumen of the cannula 66 via the port 76 of the housing 62. The drug solution further flows out from a drug solution administration port of the lumen. Accordingly, the drug solution is continuously administered to the patient.

### [Fifth Step]

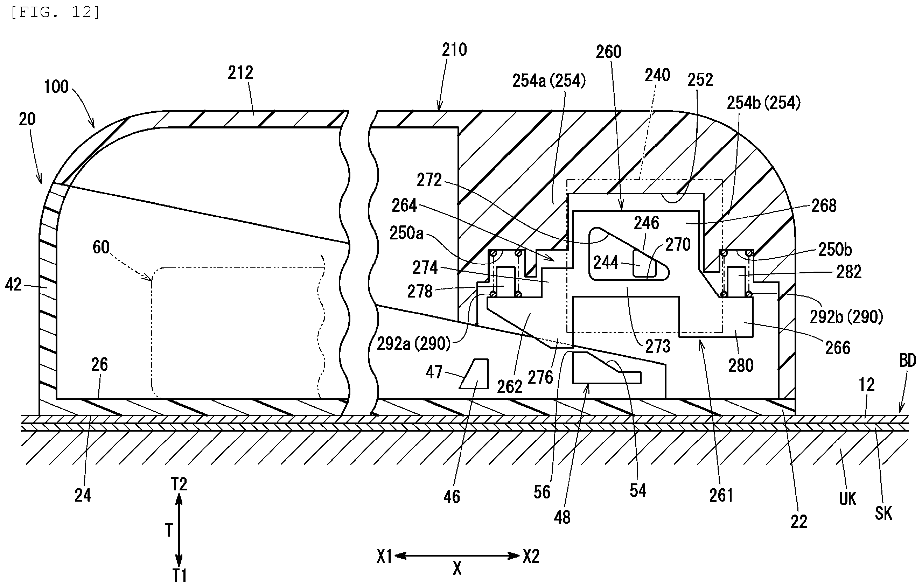

When the administration of the drug solution is completed, the user removes the drug solution administration apparatus 100 from the holder 20. First, for example, the user places a thumb on the unlocking member 240 and, as shown in FIG. 12, slides the unlocking member 240 in the detachment direction X2 with the thumb inside the insertion opening 238.

As described above, the first cam surface 246 of the cam pin 244 constituting the unlocking member 240 is attached to the second cam surface 272 of the cam hole 270 of the lock member 260. Therefore, when the unlocking member 240 moves in the detachment direction X2, the first cam surface 246 relatively slides along the second cam surface 272. The first cam surface 246 and the second cam surface 272 are inclined toward the engagement release direction T2 as the first cam surface and the second cam surface extend from the detachment direction X2 toward the attachment direction X1. Therefore, as the unlocking member 240 moves in the detachment direction X2, the lock member 260 gradually moves in the engagement release direction T2.

As shown in FIG. 12, when the unlocking member 240 moves in the detachment direction X2 with respect to the cover member 210 to some extent, the unlocking member 240 moves in the engagement release direction T2 to a position where the engagement convex portion 276 is detached from the engagement portion 56. That is, the engagement of the engagement convex portion 276 with the engagement portion 56 is released.

### [Sixth Step]

The user slides the drug solution administration apparatus 100 in this state toward the detachment direction X2 along the first inner surface 26 of the holder 20. The drug solution administration apparatus 100 moves relative to the holder 20 in the detachment direction X2 along the first inner surface 26 to be in the state shown in FIG. 12. Accordingly, the first interlock portion 74 (see FIG. 3) of the housing 62 and the second interlock portion 160 of the flow path forming member 150 are detached from each other. The engagement rib 44 (see FIG. 2) is detached from the engagement groove 136 (see FIG. 6) and relatively moves to the first concave portion 138.

As described above, the drug solution administration apparatus 100 is detached from the holder 20. As a result, in a side view from the width direction W, the end portion of the drug solution administration apparatus 100 on the detachment direction X2 protrudes from the first inner surface 26 of the holder 20 to the detachment direction X2 as in FIG. 8, and other portions of the drug solution administration apparatus 100 are placed on the holder 20. That is, the drug solution administration apparatus 100 is released from the lock state on the holder 20.

Next, when the user perceives, as a feeling, that the top portion 38 of the protection portion 34 is attached to a step formed based on the difference between the depth D1 of the preliminary concave portion 128 and the depth D2 of the accommodation concave portion 130, the user pulls up the drug solution administration apparatus 100 in the engagement release direction T2. As a result, the engagement rib 44 (see FIG. 2) is detached from the first concave portion 138 via the first insertion and removal opening 140 (see FIG. 6). The first guide rib 46 and the second guide rib 48 are detached from the second concave portion 142 via the second insertion and removal opening 144. In this way, the shielding portion 216 of the cover member 210 is prevented from interfering with the cannula unit 60.

The drug solution administration system 10 according to the present embodiment has the following effects.

As shown in FIGS. 1 to 3, the drug solution administration system 10 has the drug solution administration apparatus 100 having the reservoir 102 filled with a drug solution, and the holder 20 to which the drug solution administration apparatus 100 is detachably attached. The holder 20 is provided with the cannula 66 to be punctured into a living body.

As shown in FIGS. 7 and 8, the drug solution administration apparatus 100 has the casing 120 that accommodates the reservoir 102, the lock member 260 that is supported by the casing 120 and positions and fixes the drug solution administration apparatus 100 to the holder 20, and the elastic body 290 that biases the lock member 260. As shown in FIGS. 3 and 6, the casing 120 has the apparatus-side base portion 124 that faces the living body via the holder 20 when the drug solution administration apparatus 100 is attached to the holder 20.

As shown in FIGS. 7 and 8, the lock member 260 has the engagement convex portion 276 extending toward the holder 20. As shown in FIG. 2, the holder 20 has the holder-side base portion 22 that holds the cannula 66, and the engagement portion 56 with which the engagement convex portion 276 detachably engages. The holder-side base portion 22 has the first outer surface 24 that faces the living body when the holder 20 is fixed to the living body, and the first inner surface 26 that is a back surface of the first outer surface 24 and faces the apparatus-side base portion 124. As shown in FIGS. 3, 5, and 6, the apparatus-side base portion 124 has the second outer surface 126 facing the first inner surface 26 and the second inner surface 127 that is a back surface of the second outer surface 126 and faces the inside of the casing 120 when the drug solution administration apparatus 100 is attached to the holder 20.

As shown in FIGS. 1 to 3, a direction in which the second outer surface 126 relatively moves along the first inner surface 26 when the drug solution administration apparatus 100 is attached to the holder 20 is defined as an attachment direction X1. A direction which is opposite to the attachment direction X1 and in which the second outer surface 126 relatively moves along the first inner surface 26 when the drug solution administration apparatus 100 is detached from the holder 20 is defined as a detachment direction X2. A direction along the attachment direction X1 and the detachment direction X2 is defined as an apparatus moving direction X. A direction orthogonal to the apparatus moving direction X and in which the second inner surface 127 and the first outer surface 24 face each other is defined as a thickness direction T. One direction in the thickness direction T from the second inner surface 127 toward the first outer surface 24 is defined as an engagement direction T1. The other direction in the thickness direction T, which is a direction opposite to the engagement direction T1, is referred to as an engagement release direction T2.

In the above configuration, as shown in FIGS. 7 and 8, the lock member 260 is supported by the casing 120 to be movable in the engagement direction T1 and the engagement release direction T2. The elastic body 290 biases the lock member 260 in a state in which the engagement convex portion 276 is engaged with the engagement portion 56 toward the engagement direction T1.

According to such a configuration, a movement direction of the lock member 260 is restricted to the engagement direction T1 and the engagement release direction T2 (the thickness direction T). That is, the lock member 260 is prevented from moving in the width direction W orthogonal to the apparatus moving direction X and also orthogonal to the engagement direction T1 and the engagement release direction T2. Therefore, it is possible to avoid an increase in the size of the casing 120 along the width direction W. Accordingly, it is possible to reduce the size of the drug solution administration apparatus 100.

The lock member 260 is biased in the engagement direction T1 by the elastic body 290, so that the engagement convex portion 276 is prevented from coming off from the engagement portion 56. Therefore, even when the user or the like of the drug solution administration system 10 unintentionally touches the casing 120, a state in which the drug solution administration apparatus 100 is positioned and fixed to the holder 20 is maintained. In other words, even when a force in a direction in which the drug solution administration apparatus 100 is detached from the holder 20 acts on the casing 120 due to an unexpected situation, the drug solution administration apparatus 100 is prevented from being detached from the holder 20.

As shown in FIG. 2, the holder 20 has the holder-side guide portion 52 that guides the engagement convex portion 276 relatively moving along the attachment direction X1 to the engagement portion 56 when the drug solution administration apparatus 100 is attached to the holder 20. The holder-side guide portion 52 has a slope 54 inclined toward the engagement release direction T2 as approaching the engagement portion 56 along the attachment direction X1.

As understood by comparing FIGS. 8 to 10, when the engagement convex portion 276 moves along the holder-side guide portion 52, the lock member 260 moves relative to the casing 120 in the engagement release direction T2 in which the lock member 260 is separated from the holder 20. Accordingly, the elastic body 290 is compressed. Therefore, when the engagement convex portion 276 passes through the holder-side guide portion 52 and reaches the engagement portion 56, the elastic body 290 expands. Due to the expansion, the lock member 260 is biased toward the holder 20, and the engagement convex portion 276 engages with the engagement portion 56 as shown in FIG. 11.

Therefore, when the engagement convex portion 276 is detached from the engagement portion 56, the elastic body 290 needs to be compressed. In other words, unless the elastic body 290 is compressed, the engagement convex portion 276 is continuously prevented from coming off from the engagement portion 56. Therefore, the drug solution administration apparatus 100 is further prevented from being detached from the holder 20.

As shown in FIG. 8, the casing 120 has the casing-side guide portion 254. The casing-side guide portion 254 guides the movement of the lock member 260 in the engagement direction T1 or the engagement release direction T2.

The casing-side guide portion 254 further restricts the movement of the lock member 260 in a direction other than the engagement direction T1 and the engagement release direction T2. Therefore, the casing 120 is further prevented from increasing in size along the width direction W.

The drug solution administration apparatus 100 has the unlocking member 240 provided movably with respect to the casing 120. As shown in FIG. 12, when the lock member 260 moves in the engagement release direction T2 based on the movement of the unlocking member 240, the engagement convex portion 276 is detached separated from the engagement portion 56.

In this way, the user can easily detach the drug solution administration apparatus 100 from the holder 20 by operating the unlocking member 240.

As shown in FIGS. 7 and 8, the unlocking member 240 has the cam pin 244 including the first cam surface 246, and the lock member 260 has the second cam surface 272 with which the first cam surface 246 slides. The first cam surface 246 and the second cam surface 272 convert the movement of the unlocking member 240 relative to the casing 120 in the detachment direction X2 into the movement of the lock member 260 in the engagement release direction T2.

By moving the unlocking member 240 in the detachment direction X2 in which the drug solution administration apparatus 100 is detached from the holder 20, the engagement convex portion 276 is detached from the engagement portion 56. As understood from this, in this case, the detachment direction X2 of the drug solution administration apparatus 100 from the holder 20 coincides with a direction in which the unlocking member 240 is moved to detach the drug solution administration apparatus 100 from the holder 20. Therefore, the user can easily recognize the movement direction of the unlocking member 240 for unlocking.

Each of the first cam surface 246 and the second cam surface 272 is a slope that is inclined toward the engagement release direction T2 from the detachment direction X2 toward the attachment direction X1.

By moving the unlocking member 240 in the detachment direction X2, the engagement convex portion 276 of the lock member 260 can be easily detached from the engagement portion 56.

As shown in FIG. 1, the casing 120 has the base member 122 including the apparatus-side base portion 124, and the cover member 210 interlocked to the base member 122. As shown in FIG. 6, the base member 122 has the peripheral wall 134 that is bent toward the engagement release direction T2 with respect to the apparatus-side base portion 124.

The cover member 210 has the cover wall 214 that covers the peripheral wall 134 from the outside. The cover wall 214 has the outer wall portion 232 forming an outer surface of the cover wall 214, the inner wall portion 234 located inward of the outer wall portion 232, and the pocket-shaped storage space 236 formed between the inner wall portion 234 and the outer wall portion 232. The lock member 260 is stored in the storage space 236.

The lock member 260 is located in the storage space 236 between the inner wall portion 234 and the outer wall portion 232. Therefore, the lock member 260 is prevented from being exposed in the width direction W of the casing 120. Accordingly, it is possible to reduce the size of the drug solution administration apparatus 100.

The lock member 260 has the main body portion 264 and the leg 261 protruding from an end portion of the main body portion 264 in the apparatus moving direction X and extending in the engagement direction T1. The engagement convex portion 276 is provided on the leg 261.

Since the leg 261 extends in the engagement direction T1, the engagement convex portion 276 can be provided at a position where the leg 261 can easily engage with the engagement portion 56. That is, according to the configuration, the engagement convex portion 276 easily engages with the engagement portion 56.

The leg 261 has a first leg 262 protruding from an end portion of the main body portion 264 in the attachment direction X1 and a second leg 266 protruding from an end portion of the main body portion 264 in the detachment direction X2. The elastic body 290 has the first elastic body 292a that biases the first leg 262 in the engagement direction T1 and the second elastic body 292b that biases the second leg 266 in the engagement direction T1. The engagement convex portion 276 is provided on the first leg 262 or the second leg 266.

Since the first leg 262 is biased by the first elastic body 292a and the second leg 266 is biased by the second elastic body 292b, a biasing force of the first elastic body 292a to the lock member 260 and a biasing force of the second elastic body 292b to the lock member 260 can be balanced. Therefore, for example, when the engagement convex portion 276 is guided by the holder-side guide portion 52, the lock member 260 is prevented from being inclined with respect to the engagement direction T1 or the engagement release direction T2. Therefore, the engagement convex portion 276 easily engages with the engagement portion 56.

The engagement convex portion 276 is located further on the attachment direction X1 side than the main body portion 264 and is located further on the detachment direction X2 side than the elastic body 290, in the apparatus moving direction X.

For example, the lock member 260 biased to the leg 261 by the elastic body 290 is prevented from rotating around the leg 261. That is, the posture of the lock member 260 is stabilized. Therefore, the engagement convex portion 276 can easily move toward the engagement portion 56. As a result, the engagement convex portion 276 easily engages with the engagement portion 56.

Note that, the invention is not limited to the above-described disclosure, and various configurations can be adopted without departing from the gist of the invention.

## Claims

1. A drug solution administration system comprising:
a drug solution administration apparatus having a reservoir filled with a drug solution; and a holder to which the drug solution administration apparatus is detachably attached, the holder being provided with a cannula to be punctured into a living body, wherein
the drug solution administration apparatus includes:
a casing accommodating the reservoir,
a lock member supported by the casing and configured to position and fix the drug solution administration apparatus to the holder, and
an elastic body configured to bias the lock member,
the casing has an apparatus-side base portion facing the living body via the holder when the drug solution administration apparatus is attached to the holder,
the lock member has an engagement convex portion extending toward the holder,
the holder has a holder-side base portion configured to hold the cannula and an engagement portion with which the engagement convex portion detachably engages,
the holder-side base portion has a first outer surface facing the living body when the holder is fixed to the living body, and a first inner surface that is a back surface of the first outer surface and faces the apparatus-side base portion,
the apparatus-side base portion has a second outer surface facing the first inner surface and a second inner surface that is a back surface of the second outer surface and faces an inside of the casing when the drug solution administration apparatus is attached to the holder,
when a direction in which the second outer surface relatively moves along the first inner surface when the drug solution administration apparatus is attached to the holder is defined as an attachment direction, a direction which is opposite to the attachment direction and in which the second outer surface relatively moves along the first inner surface when the drug solution administration apparatus is detached from the holder is defined as a detachment direction, a direction along the attachment direction and the detachment direction is defined as an apparatus moving direction, a direction orthogonal to the apparatus moving direction and in which the second inner surface and the first outer surface face each other is defined as a thickness direction, one direction in the thickness direction, that is a direction from the second inner surface toward the first outer surface, is defined as an engagement direction, and the other direction in the thickness direction, that is a direction opposite to the engagement direction, is defined as an engagement release direction,
the lock member is supported by the casing to be movable in the engagement direction and the engagement release direction, and
the elastic body biases the lock member in a state in which the engagement convex portion is engaged with the engagement portion toward the engagement direction.

2. The drug solution administration system according to claim 1, wherein
when the drug solution administration apparatus is attached to the holder, the holder has a holder-side guide portion configured to guide the engagement convex portion relatively moving along the attachment direction to the engagement portion, and
the holder-side guide portion has a slope inclined toward the engagement release direction as the holder-side guide portion approaches the engagement portion along the attachment direction.

3. The drug solution administration system according to claim 1 or 2, wherein
the casing has a casing-side guide portion configured to guide a movement of the lock member in the engagement direction or the engagement release direction.

4. The drug solution administration system according to claim 1 or 2, wherein
the drug solution administration apparatus has an unlocking member provided to be movable with respect to the casing, and
the lock member moves in the engagement release direction based on a movement of the unlocking member, so that the engagement convex portion is detached from the engagement portion.

5. The drug solution administration system according to claim 4, wherein
the unlocking member has a cam pin including a first cam surface, and the lock member has a second cam surface with which the first cam surface slides, and
the first cam surface and the second cam surface convert a motion of the unlocking member moving relative to the casing in the detachment direction into a motion of the lock member moving in the engagement release direction.

6. The drug solution administration system according to claim 5, wherein
each of the first cam surface and the second cam surface is a sloped surface that is inclined toward the engagement release direction from the detachment direction toward the attachment direction.

7. The drug solution administration system according to claim 1 or 2, wherein
the casing has a base member including the apparatus-side base portion and a cover member interlocked to the base member,
the base member has a peripheral wall bent toward the engagement release direction with respect to the apparatus-side base portion,
the cover member has a cover wall covering the peripheral wall from outside,
the cover wall has an outer wall portion forming an outer surface of the cover wall, an inner wall portion located inside the outer wall portion, and a pocket-shaped storage space formed between the inner wall portion and the outer wall portion, and
the lock member is stored in the storage space.

8. The drug solution administration system according to claim 1 or 2, wherein
the lock member has a main body portion, and a leg protruding from an end portion of the main body portion in the apparatus moving direction and extending in the engagement direction, and
the engagement convex portion is provided on the leg.

9. The drug solution administration system according to claim 8, wherein
the leg has a first leg protruding from an end portion of the main body portion in the attachment direction and a second leg protruding from an end portion of the main body portion in the detachment direction,
the elastic body has a first elastic body that biases the first leg in the engagement direction and a second elastic body that biases the second leg in the engagement direction, and
the engagement convex portion is provided on the first leg or the second leg.

10. The drug solution administration system according to claim 8, wherein
the engagement convex portion is located further on the attachment direction side than the main body portion and is located further on the detachment direction side than the elastic body, in the apparatus moving direction.
